(19) Europäisches Patentamt
European Patent Office
Office européen des brevets

(11) Veröffentlichungsnummer : **0 423 566 B1**

# (12) EUROPÄISCHE PATENTSCHRIFT

(45) Veröffentlichungstag der Patentschrift :
**19.05.93 Patentblatt 93/20**

(51) Int. Cl.$^5$ : **A01N 43/653,**
// (A01N43/653, 43:36)

(21) Anmeldenummer : **90119141.1**

(22) Anmeldetag : **05.10.90**

## (54) Fungizide Wirkstoffkombinationen.

(30) Priorität : **18.10.89 DE 3934714**

(43) Veröffentlichungstag der Anmeldung :
**24.04.91 Patentblatt 91/17**

(45) Bekanntmachung des Hinweises auf die Patenterteilung :
**19.05.93 Patentblatt 93/20**

(84) Benannte Vertragsstaaten :
**BE CH DE DK ES FR GB GR IT LI NL**

(56) Entgegenhaltungen :
**EP-A- 0 040 345**
**EP-A- 0 318 704**
**DE-A- 3 702 852**

(73) Patentinhaber : **BAYER AG**
**W-5090 Leverkusen 1 Bayerwerk (DE)**

(72) Erfinder : **Dutzmann, Stefan, Dr.**
**Leinenweberweg 33**
**W-4000 Düsseldorf 13 (DE)**
Erfinder : **Brandes, Wilhelm, Dr.**
**Eichendorffstrasse 3**
**W-5653 Leichlingen (DE)**
Erfinder : **Berg, Dieter, Dr.**
**Gellertweg 27**
**W-5600 Wuppertal 1 (DE)**
Erfinder : **Holmwood, Graham, Dr.**
**Krutscheider Weg 105**
**W-5600 Wuppertal 11 (DE)**
Erfinder : **Wollweber, Detlef, Dr.**
**Paul-Ehrlich-Strasse 17**
**W-5600 Wuppertal 1 (DE)**

**EP 0 423 566 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Anmeldung betrifft neue Wirkstoffkombinationen, die aus dem bekannten 1-(4-Chlorphenyl)-4,4-dimethyl-3-(1,2,4-triazol-1-yl-methyl)-pentan-3-ol einerseits und bestimmten bekannten Fungiziden andererseits bestehen und sehr gut zur Bekämpfung von Pilzen geeignet sind.

Es ist bereits bekannt geworden, daß 1-(4-Chlorphenyl)-4,4-dimethyl-3-(1,2,4-triazol-1-yl-methyl)-pentan-3-ol fungizide Potenz besitzt (vgl. EP-OS 0 040 345). Die Wirksamkeit dieses Stoffes ist gut; sie läßt jedoch bei niedrigen Aufwandmengen in manchen Fällen zu wünschen übrig.

Ferner ist schon bekannt, das 3-Cyano-4-(2-fluor-3-chlor-phenyl)-pyrrol, 3-Cyano-4-(2,4-difluor-3-chlorphenyl)-pyrrol und 3-Cyano-4-(2,3-dichlorphenyl)-1-(N-methyl-N-furfurylaminomethyl)-pyrrol zur Bekämpfung von Pilzen eingesetzt werden können (vgl. DE-OS 3 737 984 und EP-OS 0 281 731). Die Wirkung dieser Stoffe ist aber bei niedrigen Aufwandmengen ebenfalls nicht immer befriedigend.

Es wurde nun gefunden, daß die neuen Wirkstoffkombinationen aus

A) 1-(4-Chlorphenyl)-4,4-dimethyl-3-(1,2,4-triazol-1-yl-methyl)-pentan-3-ol der Formel

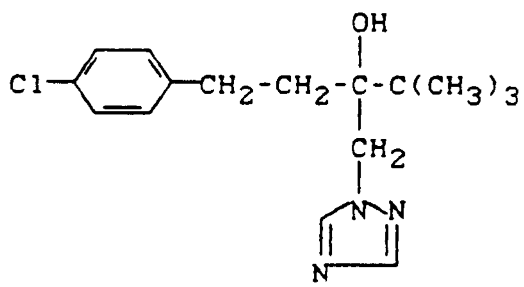

(I)

und

B) 3-Cyano-4-(2-fluor-3-chlor-phenyl)-pyrrol der Formel

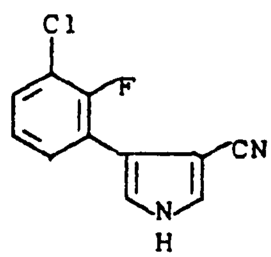

(II)

und/oder 3-Cyano-4-(2,4-difluor-3-chlorphenyl)-pyrrol der Formel

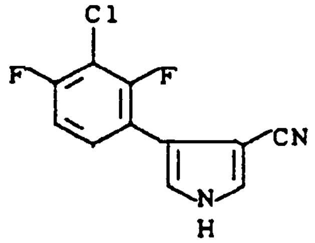

(III)

und/oder 3-Cyano-4-(2,3-dichlor-phenyl)-1-(N-methyl-N-furfuryl-aminomethyl)-pyrrol der Formel

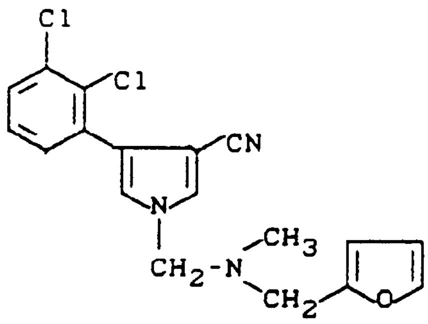

(IV)

sehr gute fungizide Eigenschaften besitzen.

Überraschenderweise ist die fungizide Wirkung der erfindungsgemäßen Wirkstoffkombinationen wesentlich höher als die Summe der Wirkungen der einzelnen Wirkstoffe. Es liegt also ein nicht vorhersehbarer echter synergistischer Effekt vor und nicht nur eine Wirkungsergänzung.

Die in den erfindungsgemäßen Wirkstoffkombinationen enthaltenen Wirkstoffe sind bereits bekannt (vgl. EP-OS 0 040 345, DE-OS 3 737 984 und EP-OS 0 281 731).

Wenn die Wirkstoffe in den erfindungsgemäßen Wirkstoffkombinationen in bestimmten Gewichtsverhältnissen vorhanden sind, zeigt sich der synergistische Effekt besonders deutliche. Jedoch können die Gewichtsverhältnisse der Wirkstoffe in den Wirkstoffkombinationen in einem relativ großen Bereich variiert werden. Im allgemeinen entfallen auf 1 Gewichtsteil an Wirkstoff der Formel (I) 0,1 bis 20 Gewichtsteile, vorzugsweise 0,2 bis 15 Gew.-Teile an Wirkstoff der Formeln (II), (III) und/oder (IV).

Die erfindungsgemäßen Wirkstoffkombinationen besitzen sehr gute fungizide Eigenschaften und lassen sich zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die erfindungsgemäßen Wirkstoffkombinationen eignen sich besonders gut zur Bekämpfung von Botrytis-Arten im Weinbau, bei Beerenobst und im Gemüsebau sowie zur Bekämpfung von Getreidekrankheiten, wie Fusarium, Pseudocercosporella, Rhizoctonia und Septoria. Als Beispiele genannt seien Fusarium nivale, Fusarium culmorum, Fusarium graminearum, Fusarium avenaceum, Pseudocercosporella herpotrichoides, Rhizoctonia cerealis, Septoria nodorum, Septoria tritici und Botrytis cinerea.

Die gute Pflanzenverträglichkeit der Wirkstoffkombinationen in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffkombinationen können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe bzw. der Wirkstoffkombinationen mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/ oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffkombinationen können in den Formulierungen in Mischung mit anderen bekannten Wirkstoffen vorliegen, wie Fungizide, Insektizide, Akarizide und Herbizide, sowie in Mischungen mit Düngemitteln oder Pflanzenwachstumsregulatoren.

Die Wirkstoffkombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver und Granulate, angewendet werden.

Die Anwendung geschieht in üblicher Weise, z.B. durch Gießen, Verspritzen, Versprüchen, Verstreuen, Verstreichen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die gute fungizide Wirkung der erfindungsgemäßen Wirkstoffkombinationen geht aus den nachfolgenden Beispiellen hervor. Während die einzelnen Wirkstoffe in der fungiziden Wirkung Schwächen aufweisen, zeigen die Kombinationen eine Wirkung, die über eine einfache Wirkungssummierung hinausgeht.

Ein synergistischer Effekt liegt bei Fungiziden immer dann vor, wenn die fungizide Wirkung der Wirkstoffkombinationen größer ist als die Summe der Wirkungen der einzeln applizierten Wirkstoffe.

## Beispiel 1

Fusarium-nivale Test (Roggen) / Saatgutbehandlung

Die Anwendung der Wirkstoffe erfolgt als Trockenbeizmittel. Sie werden zubereitet durch Abstrecken des jeweiligen Wirkstoffes bzw. der Wirkstoffkombination mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10° C und einer relativen Luftfeuchtigkeit von ca. 95 % in Saatkästen, die täglich 15 Stunden dem Licht ausgesetzt werden,

Ca. 3 Wochen nach der Aussaat erfolgt die Auswertung der Pflanzen auf Symptome des Schneeschimmels.

Um Synergismus zwischen den in diesem Versuch verwendeten Wirkstoffen aufzuzeigen, wurden die Resultate nach der von R.S. Colby beschriebenen Methode (Calculating Synergistic and Antagnostic Responses of Herbicide Combinations; Weeds 15, 20-22, 1967) ausgewertet.

Wenn

X den Wirkungsgrad, ausgedrückt in % der unbehandelten Kontrolle, beim Einsatz des Wirkstoffes A in einer Konzentration von $m$ mg Wirkstoff pro kg Saatgut,

Y den Wirkungsgrad, ausgedrückt in % der unbehandelten Kontrolle, beim Einsatz des Wirkstoffes B in einer Konzentration von $n$ mg Wirkstoff pro kg Saatgut und

E den erwarteten Wirkungsgrad, ausgedrückt in % der unbehandelten Kontrolle, beim Einsatz der Wirkstoffe A und B in Konzentrationen von $m$ und $n$ mg Wirkstoff pro kg Saatgut bedeutet,

dann ist

$$E = X + Y - \frac{X \cdot Y}{100}.$$

Ist die tatsächliche fungizide Wirkung größer als berechnet, so ist die Kombination in ihrer Wirkung überadditiv, d.h. es liegt ein synergistischer Effekt vor. In diesem Fall muß der tatsächlich beobachtete Wirkungsgrad größer sein als der aus der oben angeführten Formel errechnete Wert für den erwarteten Wirkungsgrad.

Aus der folgenden Tabelle geht eindeutig hervor, daß die gefundene Wirkung der erfindungsgemäßen Wirkstoffkombinationen größer ist als die berechnete, d.h. daß ein synergistischer Effekt vorliegt.

**Tabelle 1**

Fusarium nivale-Test (Roggen) / Saatgutbehandlung

| Wirkstoff | Wirkstoffaufwandmenge in mg/kg Saatgut | Wirkungsgrad in % der unbehandelten Kontrolle |
|---|---|---|
| **Bekannt:** | | |
| 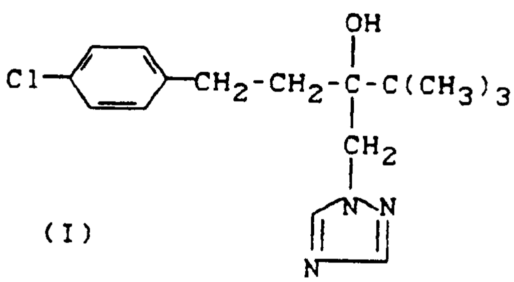 (I) | 20<br>10 | 40<br>0 |
| 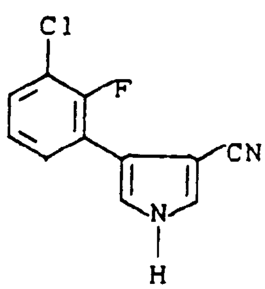 (II) | 200 | 88 |
| 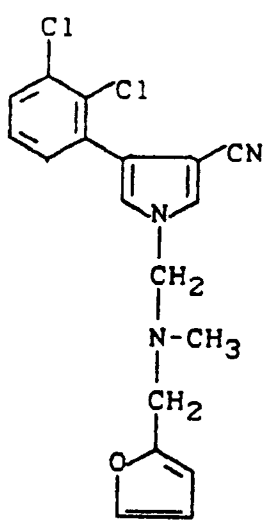 (IV) | 100 | 66 |

Tabelle 1 (Fortsetzung)

Fusarium nivale-Test (Roggen) / Saatgutbehandlung

| Wirkstoff | Wirkstoffaufwandmenge in mg/kg Saatgut | Wirkungsgrad in % der unbehandelten Kontrolle | |
|---|---|---|---|
| Erfindungsgemäß: | | gefunden | berechnet*) |
| (I) + (II) (1:10) | 20 + 200 | 100 | 92,8 |
| (I) + (IV) (1:10) | 10 + 100 | 91 | 66 |
| (unbehandelt) | - | | 0 |

*) Berechnet nach der auf der Seite 10 angegebenen Formel.

Beispiel 2

Fusarium nivale-Test (Roggen) / Saatgutbehandlung

Die Anwendung der Wirkstoffe erfolgt als Trockenbeizmittel. Sie werden zubereitet durch Abstrecken des jeweiligen Wirkstoffes bzw. der Wirkstoffkombination mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10° C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Wirkstoffe, Wirkstoffkonzentrationen und Versuchsergebnisse gehen aus der folgenden Tabelle hervor.

Tabelle 2

Fusarium nivale-Test (Roggen) / Saatgutbehandlung

| Wirkstoff | Wirkstoffaufwandmenge in mg/kg Saatgut | Wirkungsgrad in % der unbehandelten Kontrolle |
|---|---|---|
| Bekannt: | | |
| 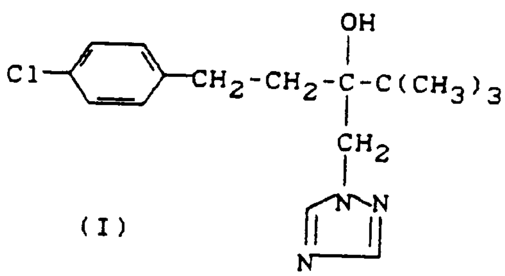 (I) | 80<br>40 | 24<br>9 |
| 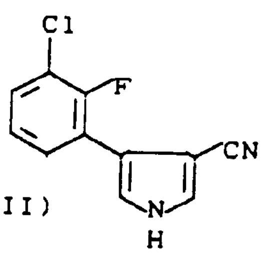 (II) | 40 | 48 |
| 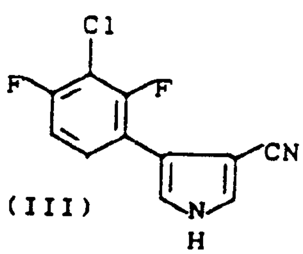 (III) | 80<br>40 | 43<br>37 |

Tabelle 2 (Fortsetzung)

Fusarium nivale-Test (Roggen) / Saatgutbehandlung

| Wirkstoff | Wirkstoffaufwandmenge in mg/kg Saatgut | Wirkungsgrad in % der unbehandelten Kontrolle |
|---|---|---|
| 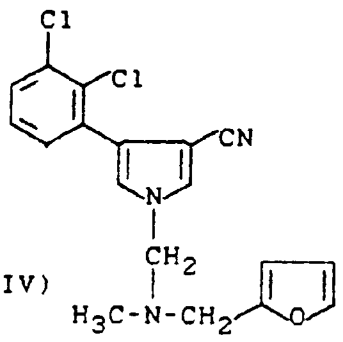 (IV) | 80 | 93 |
| unbehandelt | - | 0 |

Tabelle 2 (Fortsetzung)

Fusarium nivale-Test (Roggen) / Saatgutbehandlung

| Wirkstoff | Wirkstoffaufwandmenge in mg/kg Saatgut | Wirkungsgrad in % der unbehandelten Kontrolle |
|---|---|---|
| Erfindungsgemäß: | | |
| (I) + (II) (1:1) | 20 + 20 | 78 |
| (I) + (II) (1:7) | 5 + 35 | 93 |
| (I) + (III) (1:1) | 40 + 40 | 89 |
| (I) + (III) (1:7) | 5 + 35 | 96 |
| (I) + (IV) (1:7) | 10 + 70 | 100 |

Beispiel 3

Botrytis-Test (Bohne) / protektiv

Zur Herstellung einer zweckmäßigen Wirkstoffzubereitung vermischt man 1 Gewichtsteil Wirkstoff bzw. Wirkstoffkombination mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20° C aufgestellt. 3 Tage nach der Inokulation wird die Größe der Befallsflecken auf den Blättern ausgewertet.

Um Synergismus zwischen den in diesem Versuch verwendeten Wirkstoffen aufzuzeigen, wurden die Resultate nach der von R.S. Colby beschriebenen Methode (Calculating Synergistic and Antagonistic Responses of Herbicide Combinations; Weeds 15, 20-22, 1967) ausgewertet.

Wenn

X den Wirkungsgrad, ausgedrückt in % der unbehandelten Kontrolle, beim Einsatz des Wirkstoffes A in einer Konzentration von m ppm,

Y den Wirkungsgrad, ausgedrückt in % der unbehandelten Kontrolle, beim Einsatz des Wirkstoffes B in einer Konzentration von n ppm und

E den erwarteten Wirkungsgrad, ausgedrückt in % der unbehandelten Kontrolle, beim Einsatz der Wirkstoffe A und B in Konzentrationen von m und n ppm bedeutet,

dann ist

$$E = X + Y - \frac{X \cdot Y}{100}.$$

Ist die tatsächliche fungizide Wirkung größer als berechnet, so ist die Kombination in ihrer Wirkung überadditiv, d.h. es liegt ein synergistischer Effekt vor. In diesem Fall muß der tatsächlich beobachtete Wirkungsgrad größer sein als der aus der oben angeführten Formel errechnete Wert für den erwarteten Wirkungsgrad.

Aus der folgenden Tabelle geht eindeutig hervor, daß die gefundene Wirkung der erfindungsgemäßen Wirktoffkombinationen größer ist als die berechnete, d.h. daß ein synergistischer Effekt vorliegt.

Tabelle 3

Botrytis-Test (Buschbohne) / protektiv

| Wirkstoff | | Wirkungsgrad in % der unbehandelten Kontrolle bei einer Wirkstoffkonzentration von 20 ppm | 10 ppm |
|---|---|---|---|
| **Bekannt:** | | | |
| 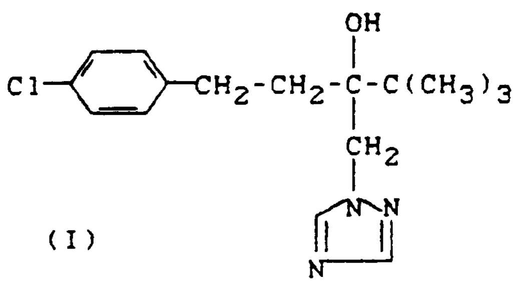 (I) | | 21 | |
| 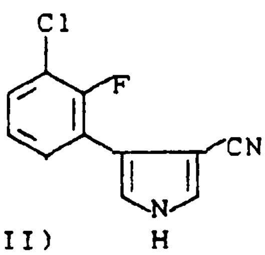 (II) | | | 10 |
| **Erfindungsgemäß:** | | gefunden | berechnet*) |
| (I) + (II) (2:1) | 20 ppm + 10 ppm | 72 | 29 |
| - (Kontrolle) | | 0 | |

*) Berechnet nach der auf der Seite 19 angegebenen Formel

Beispiel 4

Cochliobolus sativus-Test (Gerste) / protektiv

Lösungsmittel: 100 Gew.-Teile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20° C und 100° C rel. Luftfeuchtigkeit

in einer Inkubationskabine.

Die Pflanzen werden in einem Gewächshaus bei einer Temperatur von 20° C und einer relativen Luftfeuchtigkeit von ca. 80 % aufgestellt.

7 Tage nach der Inokulation erfolgt die Auswertung.

Wirkstoffe, Wirkstoffkonzentrationen und Versuchsergebnisse gehen aus der folgenen Tabelle hervor.

Tabelle 4

Cochliobolus sativus-Test (Gerste) / protektiv

| Wirkstoff | Wirkstoffkonzentration in der Spritzbrühe in ppm | Wirkungsgrad in % der unbehandelten Kontrolle |
|---|---|---|
| Bekannt: | | |
| 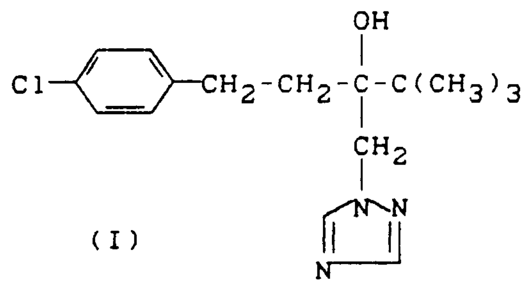 (I) | 25<br>12,5 | 58<br>43 |
| 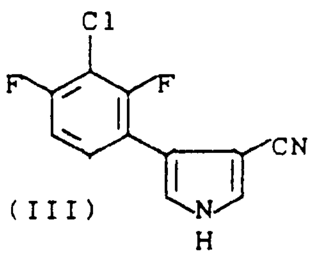 (III) | 12,5 | 0 |
| 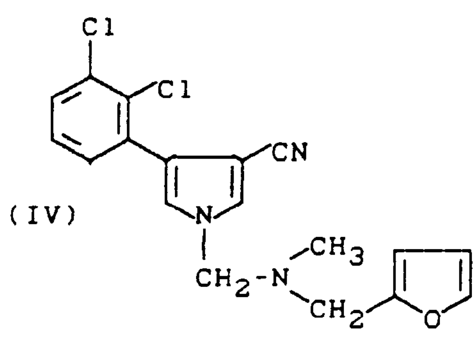 (IV) | 25 | 43 |

Tabelle 4 (Fortsetzung)

Cochliobolus sativus-Test (Gerste) / protektiv

| Wirkstoff | Wirkstoffkonzentration in der Spritzbrühe in ppm | Wirkungsgrad in % der unbehandelten Kontrolle |
|---|---|---|
| Erfindungsgemäß: | | |
| (I) + (III) (1:1) | 6,25 + 6,25 | 58 |
| (I) + (IV) (1:1) | 12,5 + 12,5 | 72 |

Beispiel 5

Pyrenophora teres-Test (Gerste) / protektiv

Lösungsmittel: 100 Gew.-Teile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20° C und 100° C rel. Luftfeuchtigkeit in einer Inkubationskabine.

Die Pflanzen werden in einem Gewächshaus bei einer Temperatur von 20° C und einer relativen Luftfeuchtigkeit von ca. 80 % aufgestellt.

7 Tage nach der Inokulation erfolgt die Auswertung.

Wirkstoffe, Wirkstoffkonzentrationen und Versuchsergebnisse gehen aus der folgenen Tabelle hervor.

Tabelle 5

Pyrenophora teres-Test (Gerste) / protektiv

| Wirkstoff | Wirkstoffkonzentration in der Spritzbrühe in ppm | Wirkungsgrad in % der unbehandelten Kontrolle |
|---|---|---|
| Bekannt: | | |
| 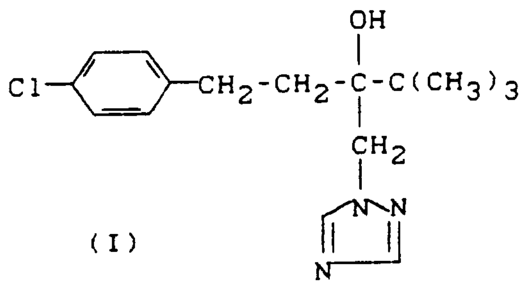 (I) | 12,5 | 50 |
| 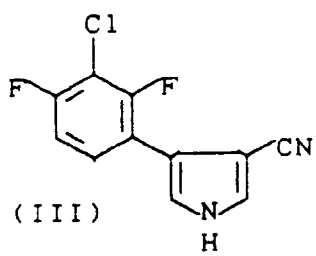 (III) | 12,5 | 0 |
| Erfindungsgemäß: | | |
| (I) + (III) (1:1) | 6,25 + 6,25 | 75 |

**Patentansprüche**

1. Fungizide Mittel, gekennzeichnet durch einen Gehalt an einer Wirkstoffkombination bestehend aus
   A) 1-(4-Chlorphenyl)-4,4-dimethyl-3-(1,2,4-triazol-1-yl-methyl)-pentan-3-ol der Formel

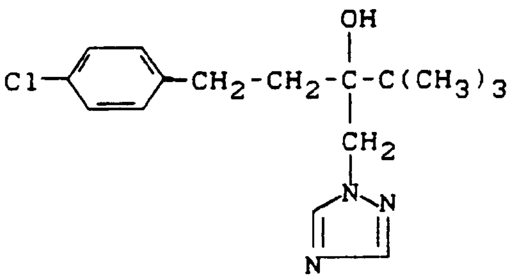

(I)

und
B) 3-Cyano-4-(2-fluor-3-chlor-phenyl)-pyrrol der Formel

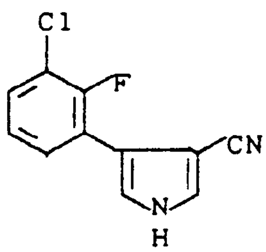

(II)

und/oder 3-Cyano-4-(2,4-difluor-3-chlor-phenyl)-pyrrol der Formel

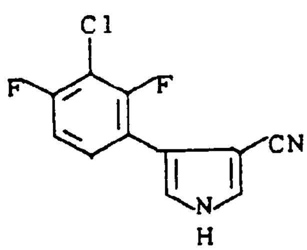

(III)

und/oder 3-Cyano-4-(2,3-dichlor-phenyl)-1-(N-methyl-N-furfuryl-aminomethyl)-pyrrol der Formel

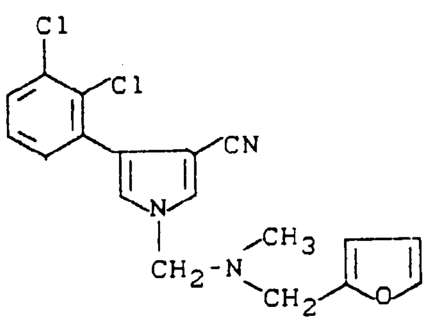

(IV)

.

2. Mittel gemäß Anspruch 1, dadurch gekennzeichnet, daß in den Wirkstoffkombinationen das Gewichtsverhältnis von Wirkstoff der Formel (I) zu Wirkstoff der Formeln (II), (III) und/oder (IV) zwischen 1:0,1 und 1:20 liegt.

3. Verfahren zur Bekämpfung von Pilzen, dadurch gekennzeichnet, daß man Wirkstoffkombinationen gemäß Anspruch 1 auf die Pilze und/oder deren Lebensraum einwirken läßt.

4. Verwendung von Wirkstoffkombinationen gemäß Anspruch 1 zur Bekämpfung von Pilzen.

5. Verfahren zur Herstellung von fungiziden Mitteln, dadurch gekennzeichnet, daß man Wirkstoffkombinationen gemäß Anspruch 1 mit Streckmitteln und/oder oberflächenaktiven Stoffen vermischt.

## Claims

1. Fungicidal agents, characterized in that they contain a combination of active compounds, consisting of
A) 1-(4-chlorophenyl)-4,4-dimethyl-3-(1,2,4-triazol-1-yl-methyl)-pentan-3-ol, of the formula

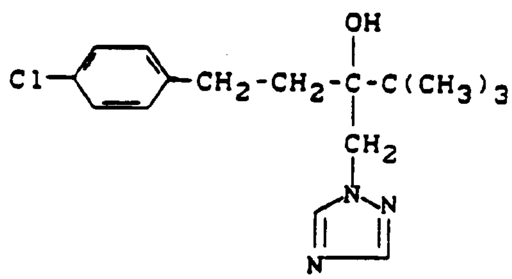

(I),

and
B) 3-cyano-4-(2-fluoro-3-chloro-phenyl)-pyrrole, of the formula

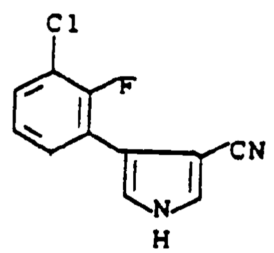

(II),

and/or 3-cyano-4-(2,4-difluoro-3-chlorophenyl)-pyrrole, of the formula

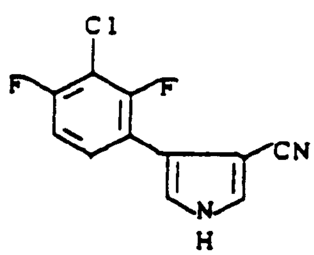

(III),

and/or 3-cyano-4-(2,3-dichloro-phenyl)-1-(N-methyl-N-furfuryl-aminomethyl)-pyrrole, of the formula

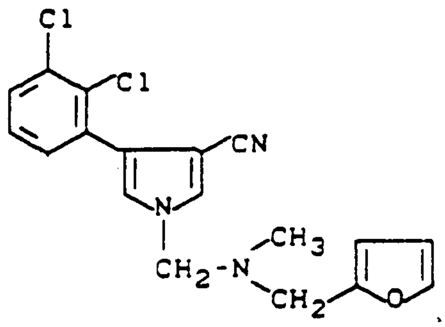

(IV).

2. Agents according to Claim 1, characterized in that the weight ratio, in the combinations of active compounds, of the active compound of the formula (I) to the active compound of the formulae (II), (III) and/or (IV) is between 1:0.1 and 1:20.

3. Method for combating fungi, characterized in that combinations of active compounds according to Claim 1 are allowed to act on the fungi and/or their environment.

4. Use of combinations of active compounds according to Claim 1 for combating fungi.

5. Process for the preparation of fungicidal agents, characterized in that combinations of active compounds according to Claim 1 are mixed with extenders and/or surface-active substances.

**Revendications**

1. Produits fongicides, caractérisés en ce qu'ils contiennent une combinaison de substances actives consistant en

A) le 1-(4-chlorophényl)-4,4-diméthyl-3-(1,2,4-triazole-1-ylméthyl)-pentane-3-ol de formule :

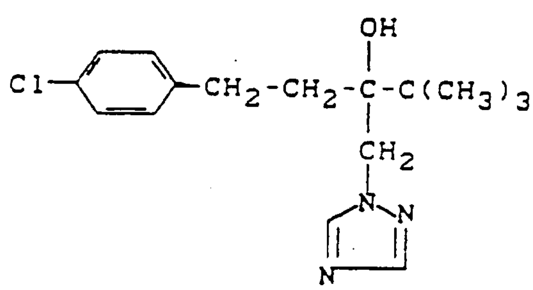

(I)

et

B) le cyano-4-(2-fluoro-3-chlorophényl)-pyrrole de formule :

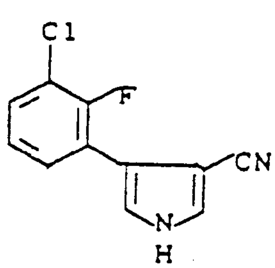

(II)

et/ou le 3-cyano-4-(2,4-difluoro-3-chlorophényl)-pyrrole de formule :

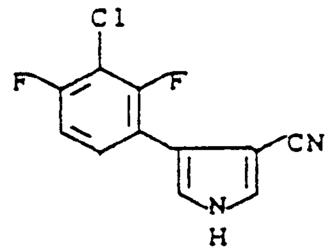

(III)

et/ou le 3-cyano-4-(2,3-dichlorophényl)-1-(N-méthyl-N-furfurylaminométhyl)-pyrrole de formule :

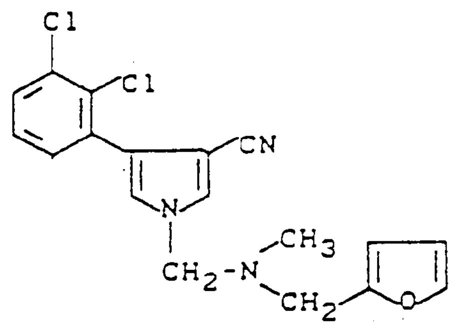

(IV)

2. Produit selon la revendication 1 caractérisé en ce que, dans les combinaisons de substances actives, les proportions relatives en poids entre la substance active de formule (I) et la substance active de formules (II), (III) et/ou (IV) vont de 1:0,1 à 1:20.

3. Procédé pour combattre les mycètes, caractérisé en ce que l'on fait agir des combinaisons de substances actives selon la revendication 1, sur les mycètes et/ou leur habitat.

4. Utilisation des combinaisons de substances actives selon la revendication 1, pour la lutte contre les mycètes.

5. Procédé de préparation de produits fongicides, caractérisé en ce que l'on mélange des combinaisons de substances actives selon la revendication 1, avec des diluants et/ou des agents tensioactifs.